# EUROPEAN PATENT SPECIFICATION

(11) **EP 4 076 209 B1**
(45) Date of publication and mention of the grant of the patent: **04.09.2024**
(21) Application number: 20842818.5
(22) Date of filing: 18.12.2020
(51) Int. Cl.: A61B 8/08, A61B 8/00

(54) **NEEDLE STERILITY BREACH WARNING USING MAGNETIC NEEDLE TRACKING**
NADELSTERILITÄTSWARNUNG MITTELS MAGNETISCHER NADELVERFOLGUNG
ALERTE DE VIOLATION DE STÉRILITÉ D'AIGUILLE À L'AIDE D'UN SUIVI D'AIGUILLE MAGNÉTIQUE

(30) Priority: 19.12.2019 US 201962950859 P
(43) Date of publication of application: 26.10.2022
(60) Divisional application: 24175400.1
(73) Proprietor: Bard Access Systems, Inc., Salt Lake City, UT 84116 (US)
(72) Inventor: PRINCE, Matthew, J., Herriman, UT 84096 (US)
(74) Representative: Hoffmann Eitle
(86) International application number: PCT/US2020/066186
(87) International publication number: WO 2021/127545

(56) References cited:
- EP-A2- 2 913 000
- WO-A1-2017/079732
- KR-A- 20100 047 436
- US-A1- 2012 143 029
- US-A1- 2012 143 029
- US-A1- 2016 000 399
- US-A1- 2018 310 955
- US-A1- 2019 216 423

## Description

### SUMMARY OF THE INVENTION

The invention is defined by an ultrasound system according to claim 1.

### SUMMARY OF THE DISCLOSURE

Briefly summarized, embodiments disclosed herein are directed to systems, methods and apparatuses directed to determining proximity of a needle tip to an ultrasound probe head and to provide an indication when the needle tip is moved within a threshold distance of the probe in order to protect against the needle tip from breaching a probe head cover, therefore preserving sterility of the needle insertion process.

Ultrasound imaging systems are used to facilitate vascular access under ultrasound image guidance. To improve acoustic communication between the transducer located in the ultrasound probe head and the skin surface of the patient, the probe head often includes various additional structures, for example covers, hydrogel spacers, and the like. Such structures are optimized for conveying acoustic energy and as such, are not necessarily formed of materials that are resistant to damage from accidental needle sticks. Needles for accessing the vasculature are inserted adjacent to the position of the ultrasound probe head as it is held against the skin surface of the patient. This is often performed while the clinician is observing a console or display disposed remotely from the insertion site. Accordingly, there is a risk that the needle can be accidentally inserted into the probe head, or associated structures, causing significant damage to the probe head, transducer, or associated structures.

Due to the risk that the needle tip may accidentally be inserted into the probe head or associated structures, there has recently been calls within the industry to preserve sterility of the needle insertion process by achieving a high level of disinfection on the ultrasound probe between every ultrasound procedure. However, achieving a high level of disinfection is a costly method for preserving sterility from both monetary and time perspectives. As used herein, the terms "disinfect" and "cleanse" should be understood to mean cleaning so as to destroy or prevent the growth of microorganisms.

For instance, a high level of disinfection may be achieved through the use of an array of light sources that apply disinfecting light in a high energy visible light wavelength range. However, a system that deploys such an array of light sources may have certain disadvantages based on the setting. For example, such a system may be expensive and thus prohibitive for many medical facilities or institutions to purchase. Additionally, such a system may potentially be large and/or cumbersome, making use of the system frustrating and time consuming, especially if a medical professional is required to transport components to the system after each use (e.g., detach a probe head from a catheter placement system and transport the probe head to the location of the system deploying the array of light sources). In other situations, various chemicals may be used to achieve a high level of disinfection. However, the use of chemicals may be harmful to the components being disinfected. EP 2 913 000 is directed to an integrated catheter placement system configured for accurately placing a catheter within the vasculature of a patient comprising a system console including a control processor, a tip location sensor for temporary placement on a portion of a body of the patient, and an ultrasound probe. The tip location sensor senses a magnetic field of a stylet disposed in a lumen of the catheter when the catheter is disposed in the vasculature.

Disclosed herein is an ultrasound system according to claim 1.

In some embodiments, the ultrasound system includes a console apparatus that includes the non-transitory computer-readable medium, the console apparatus communicatively coupled to the probe.

In some embodiments, the magnetic sensor includes a sensor array including a plurality of sensors. In some embodiments, the logic, when executed by the one or more processors, causes performance of additional operations including determining a positioning of the needle in three spatial dimensions including X, Y, Z coordinate space. In some embodiments, the logic further detects a pitch attitude and a yaw attitude of the needle.

In some embodiments, the non-transitory computer-readable medium is included within the probe. In some embodiments, the needle is magnetized and the magnetic field is generated by the magnetized needle.

In some embodiments, the magnetic sensor is located at a distal end of the probe. In some embodiments, determining the distance between the distal tip of the needle and the probe based on the strength of the magnetic field. In some embodiments, the magnetic sensor is a three-axis sensor configured to detect corresponding orthogonal components of the magnetic field.

These and other features of the concepts provided herein will become more apparent to those of skill in the art in view of the accompanying drawings and following description, which disclose particular embodiments of such concepts in greater detail.

### BRIEF DESCRIPTION OF THE DRAWINGS

Embodiments of the disclosure are illustrated by way of example and not by way of limitation in the figures of the accompanying drawings, in which like references indicate similar elements and in which:
FIG. 1 illustrates a block diagram depicting various elements of an ultrasound-based guidance system for needles and other medical components according to some embodiments;
FIG. 2 illustrates a simplified view of a patient and a catheter being inserted therein, showing one possible environment in which the guidance system of FIG. 1 is deployed according to some embodiments;
FIG. 3A illustrates a top view of the ultrasound probe of the guidance system of FIG. 1 according to some embodiments;
FIG. 3B illustrates a sensor array for attachment to an ultrasound probe of the guidance system of FIG. 1 according to some embodiments;
FIG. 4 illustrates a first view of an ultrasound probe as a needle is advanced toward a target insertion site of a patient's skin according to some embodiments;
FIG. 5 is a flowchart illustrating an exemplary method for detecting proximity of a needle to a probe using the guidance system of FIG. 1 according to some embodiments;
FIG. 6 illustrates a second view of an ultrasound probe as a needle is advanced toward a target insertion site of a patient's skin according to some embodiments;
FIGS. 7A-7B illustrate a flowchart showing an exemplary method for detecting proximity of a needle to a needle shield affixed to a probe using the guidance system of FIG. 1 according to some embodiments;
FIG. 8 illustrates a side view of a needle for use with the guidance system of FIG. 1 according to some embodiments;
FIG. 9 illustrates a simplified view of an ultrasound probe and needle including elements of an electromagnetic signal-based guidance system according to some embodiments; and
FIG. 10 illustrates a simplified view of an ultrasound probe and needle including elements of an electromagnetic signal-based guidance system according to some embodiments.

### DETAILED DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal end portion" of, for example, a probe disclosed herein includes a portion of the probe intended to be near a clinician when the probe is used on a patient. Likewise, a "proximal length" of, for example, the probe includes a length of the probe intended to be near the clinician when the probe is used on the patient. A "proximal end" of, for example, the probe includes an end of the probe intended to be near the clinician when the probe is used on the patient. The proximal portion, the proximal end portion, or the proximal length of the probe can include the proximal end of the probe; however, the proximal portion, the proximal end portion, or the proximal length of the probe need not include the proximal end of the probe. That is, unless context suggests otherwise, the proximal portion, the proximal end portion, or the proximal length of the probe is not a terminal portion or terminal length of the probe.

With respect to "distal," a "distal portion" or a "distal end portion" of, for example, a probe disclosed herein includes a portion of the probe intended to be near or in a patient when the probe is used on the patient. Likewise, a "distal length" of, for example, the probe includes a length of the probe intended to be near or in the patient when the probe is used on the patient. A "distal end" of, for example, the probe includes an end of the probe intended to be near or in the patient when the probe is used on the patient. The distal portion, the distal end portion, or the distal length of the probe can include the distal end of the probe; however, the distal portion, the distal end portion, or the distal length of the probe need not include the distal end of the probe. That is, unless context suggests otherwise, the distal portion, the distal end portion, or the distal length of the probe is not a terminal portion or terminal length of the probe. Finally, unless defined otherwise, all technical and scientific terms used herein have the same meaning as commonly understood by those of ordinary skill in the art.

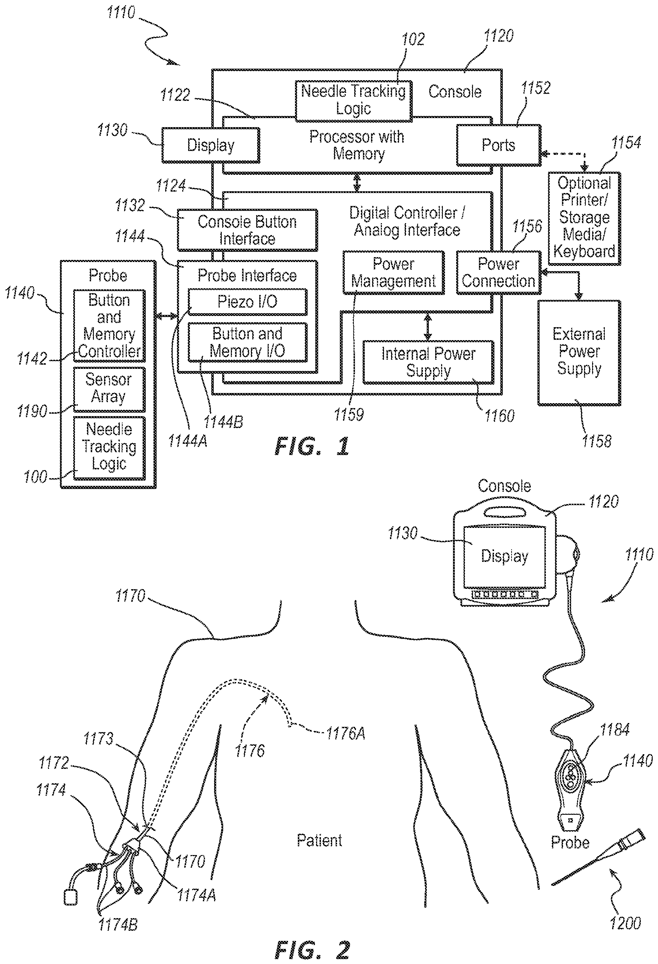

Referring to now FIGS. 1-2, the illustrations depict various components of a catheter placement system or alternatively referred to as an ultrasound-based guidance system ("guidance system"), generally designated at 1110, configured in accordance with one example embodiment of the present invention. As shown, the system 1110 generally includes a console 1120, display 1130, probe 1140, and sensor 1150, each of which is described in further detail below.

Specifically, FIG. 2 illustrates the general relation of these components to a patient 1170 during a procedure to place a catheter 1172 into the patient vasculature through a skin insertion site 1173. FIG. 2 shows that the catheter 1172 generally includes a proximal portion 1174 that remains exterior to the patient and a distal potion 1176 that resides within the patient vasculature after placement is complete. The system 1110 is employed to ultimately position a distal tip 1176A of the catheter 1172 in a desired position within the patient vasculature. In one embodiment, the desired position for the catheter distal tip 1176A is proximate the patient's heart, such as in the lower one-third (1/3^{rd}) portion of the Superior Vena Cava ("SVC"). Of course, the system 1110 can be employed to place the catheter distal tip in other locations. The catheter proximal portion 1 1 74 further includes a hub 1174A that provides fluid communication between the one or more lumens of the catheter 1172 and one or more extension legs 1174B extending proximally from the hub.

A processor 1122, including non-volatile memory such as EEPROM for instance, is included in the console 1120 for controlling system function during operation of the system 1110, thus acting as a control processor. A digital controller/analog interface 1124 is also included with the console 1120 and is in communication with both the processor 1122 and other system components to govern interfacing between the probe 1140, sensor 1150, and other system components.

The system 1110 further includes ports 1152 for connection with the sensor 1150 and optional components 1154 including a printer, storage media, keyboard, etc. The ports in one embodiment are USB ports, though other port types or a combination of port types can be used for this and the other interfaces connections described herein. A power connection 1156 is included with the console 1120 to enable operable connection to an external power supply 1158. An internal battery 1160 can also be employed, either with or exclusive of an external power supply. Power management circuitry 1159 is included with the digital controller/analog interface 1124 of the console to regulate power use and distribution. The display 1130 in the present embodiment is integrated into the console 1120 and is used to display information to the clinician during the catheter placement procedure. In another embodiment, the display may be separate from the console.

FIG. 1 further shows that, in one embodiment, the probe 1140 may include a button and memory controller 1142 for governing button and probe operation and a sensor array 1190. The button and memory controller 1142 can include non-volatile memory, such as EEPROM, in one embodiment. The button and memory controller 1142 is in operable communication with a probe interface 1144 of the console 1120, which includes a piezo input/output component 1144A for interfacing with the probe piezoelectric array and a button and memory input/output component 1144B for interfacing with the button and memory controller 1142.

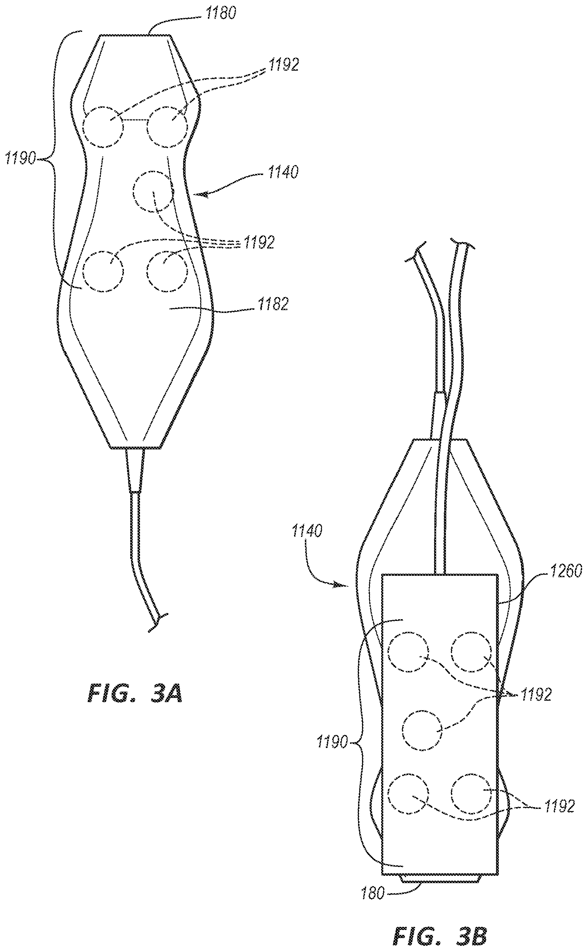

Referring to FIG. 3A, a top view of the ultrasound probe of the guidance system of FIG. 1 is shown according to some embodiments. The probe 1140 is employed in connection with ultrasound-based visualization of a vessel, such as a vein, in preparation for insertion of the needle 1200 and/or catheter 1172 into the vasculature. Such visualization gives real-time ultrasound guidance and assists in reducing complications typically associated with such introduction, including inadvertent arterial puncture, hematoma, pneumothorax, etc.

The handheld probe 1140 includes a head 1180 that houses a piezoelectric array for producing ultrasonic pulses and for receiving echoes thereof after reflection by the patient's body when the head is placed against the patient's skin proximate the prospective insertion site 1173 (FIG. 2). The probe 1140 further includes a plurality of control buttons 1184 (FIG. 2) for controlling the system, thus eliminating the need for the clinician to reach out of the sterile field, which is established about the patient insertion site prior to establishment of the insertion site, to control the system 1110.

As seen in FIG. 3A, the probe 1140 includes a sensor component, such as the sensor array 1190, for detecting the position, orientation, and movement of the needle 1200 during ultrasound imaging procedures, such as those described above. As will be described in further detail below, the sensor array includes a plurality of magnetic sensors 1192 embedded within the housing of the probe. The sensors 1192 are configured to detect a magnetic field associated with the needle 1200 and enable the system 1110 to track the needle. Though configured here as magnetic sensors, it is appreciated that the sensors 1192 can be sensors of other types and configurations, as will be described. Also, though they are shown in FIG. 3A, as included with the probe 1140, the sensors 1192 of the sensor array 1190 can be included in a component separate from the probe, such as a separate handheld device. In the present embodiment, the sensors 1192 are disposed in a planar configuration below a top face 1182 of the probe 1140, though it is appreciated that the sensors can be arranged in other configurations, such as in an arched or semi-circular arrangement.

In one embodiment, each of the sensors 1192 includes three orthogonal sensor coils for enabling detection of a magnetic field in three spatial dimensions. Such three dimensional ("3-D") magnetic sensors can be purchased, for example, from Honeywell Sensing and Control of Morristown, NJ. Further, the sensors 1192 in one embodiment are configured as Hall-effect sensors, though other types of magnetic sensors could be employed. Further, instead of 3-D sensors, a plurality of one dimensional magnetic sensors can be included and arranged as desired to achieve 1-, 2-, or 3-D detection capability.

In the illustrated embodiment, five sensors 1192 are included in the sensor array 1190 so as to enable detection of the needle 1200 in not only the three spatial dimensions (i.e., X, Y, Z coordinate space), but also the pitch and yaw attitude of the needle itself. Note that in one embodiment, orthogonal sensing components of two or more of the sensors 1192 enable the pitch and yaw attitude of the needle 1200 to be determined. In other embodiments, fewer or more sensors can be employed in the sensor array. More generally, it is appreciated that the number, size, type, and placement of the sensors of the sensor array can vary from what is explicitly shown here.

Referring to FIG. 3B, a sensor array for attachment to an ultrasound probe of the guidance system of FIG. 1 is shown according to some embodiments. It is appreciated that in one embodiment the sensor array need not be incorporated natively into the ultrasound imaging device, but can be included therewith in other ways. FIG. 3B shows one example of this, wherein an attachable sensor module 1260 including the sensors 1192 of the sensor array 1190 is shown attached to the ultrasound probe 1140. Such a configuration enables needle guidance as described herein to be achieved in connection with a standard ultrasound imaging device, *i.e.,* a device not including a sensor array integrated into the ultrasound probe or a processor and algorithms configured to locate and track a needle as described above. As such, the sensor module 1260 in one embodiment includes a processor and algorithms suitable for locating and tracking the needle or other medical component and for depicting on a display the virtual image of the needle for overlay on to the ultrasound image. In one embodiment, the sensor module 1260 can be included with a module display 1262 for depiction of the needle tracking. These and other configurations of the guidance system are therefore contemplated.

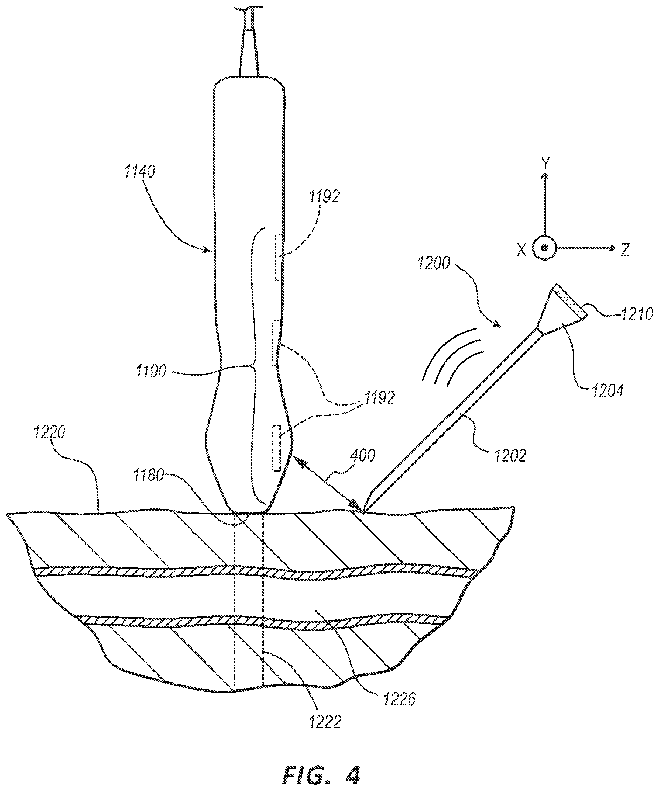

Referring to FIG. 4, a simplified view of the ultrasound probe of the guidance system being used to guide a needle including an electromagnetic component toward a vessel within the body of a patient and determine a distance between a distal tip of the needle and the ultrasound probe is shown according to some embodiments. The illustration shows the ultrasound probe 1140 of the system 1110 and the needle 1200 in position and ready for insertion thereof through a skin surface 1220 of a patient to access a targeted internal body portion (e.g., the vessel 1226). In particular, the probe 1140 is shown with its head 1180 placed against the patient skin and producing an ultrasound beam 1222 so as to ultrasonically image a portion of a vessel 1226 beneath the patient skin surface 1220. Further, the needle 1200 is shown to generate a magnetic field that is detectable by the sensor array 1190 of the probe 1140.

The magnetic field may be generated in any one of a variety of ways. In one embodiment, the cannula of the needle 1200 may be comprised of a material of relatively high magnetic permeability, such as stainless steel, or other suitable needle cannula material susceptible to magnetization. For instance, the system 1100 may further include a needle guard (not shown), which includes a hollow cylindrical body that defines a cavity, or cylindrical volume, into which the cannula is removably inserted. Disposal of the cannula within the cylindrical volume for a suitable amount of time may cause a magnetic material of an outer sleeve of the needle guard to magnetize the cannula such that it possesses a magnetic field and can be detected and tracked by the guidance system disclosed herein.

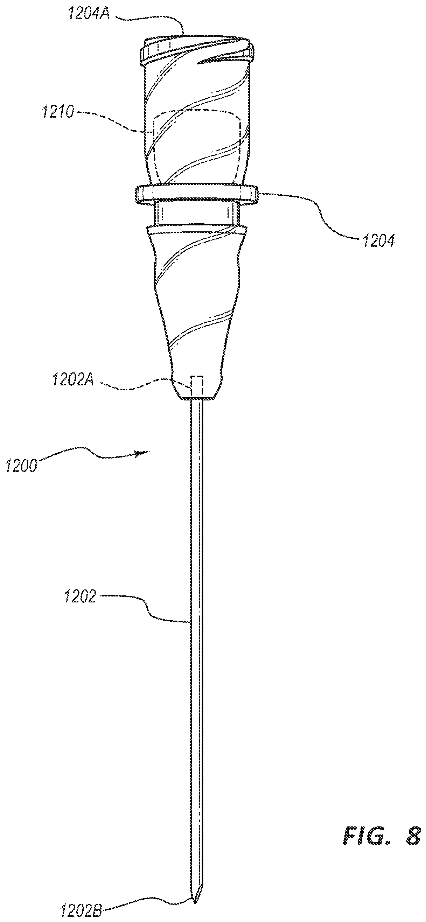

In an alternative embodiment, for example, one or more magnetic elements may be disposed more proximally along the stylet length or may be included within a hub (FIG. 8). It is appreciated that the stylet can be configured in one of many different ways, analogous examples of which can be found in U. S. Pat. No. 5,099,845 titled "Medical Instrument Location Means," and. U.S. Pat. No. 8,784,336, titled "Stylet Apparatuses and Methods of Manufacture." These and other variations are therefore contemplated.

As mentioned above, the system 1110 in the present embodiment may be configured to detect the position, orientation, and movement of the needle 1200 described above. In particular, the sensor array 1190 of the probe 1140 is configured to detect a magnetic field generated by or otherwise associated with the needle 1200. In some embodiments, each of the sensors 1192 of the sensor array 1190 may be configured to spatially detect the magnetic field in three dimensional space. Thus, during operation of the system 1110, magnetic field strength data of the needle 1200 sensed by each of the sensors 1192 is forwarded to a processor, such as the processor 1122 of the console 1120 (FIG. 1), which, in conjunction with logic, such as the needle tracking logic 102 of the console 1120 (FIG. 1) computes, in real-time, the position and/or orientation of the needle 1200 as well as the distance 400 between the distal tip of the needle 1200 and the probe 1140.

For example, the needle tracking logic 102 is stored on the memory 1122 of the console 1120 that, upon execution by the processor 1122, performs operations resulting in the determination of the position of the needle 1200, details of which are described below. Based on the determination of the position of the needle 1200 and optionally in conjunction with the length of the needle 1200 and the positioning of each sensor 1192 of the sensor array 1190, the needle tracking logic 102 may determine the distance 400 between the probe 1140 and the distal tip of the needle 1200. Specifically, the distance between the probe 1140 and the distal tip of the needle 1200 is determined based on the magnetic field strength detected by the sensor array 1190.

When the distance 400 between the probe 1140 and the distal tip of the needle 1200 is within a predetermined threshold, the system 1110 generates an alert. The alert may take several forms such as visual or audible cues. The visual cues may be any assortment of colors and/or shapes that may be displayed on the console display 1130, on a display or indicator elements (e.g., LEDs) of the probe 1140 (not shown), or on any other display device. Similarly, the audible cues may be provided via speakers (not shown) of the console 1120 and/or external speakers coupled to the console 1120, e.g., via a port 1152. The audible cues may take any form, examples of which may include, but are not limited to, a series of beeps, volume control of a steady sound (e.g., increasing/decreasing volume based on the proximity of the needle 1200 to the probe 1140), etc. Additionally, or as an alternative, the alerts may be provided as haptic feedback. For example, the probe 1140 may include a taptic engine (not shown) that receives a signal from the console 1120 and, in response, provides haptic feedback indicating a proximity of the needle 1200 to the probe 1140. The haptic feedback may be provided by other devices such as smart wearable devices (e.g., a "smart" watch that operates as an internet of things (IoT) device).

Additionally, multiple predetermined thresholds may be implemented by the system 1110 such that varying alerts are generated based on the corresponding threshold. As the needle 1200 advances toward the probe 1140 and crosses varying thresholds, the alerts generated for each progressive threshold may become more and more cautionary. For example, a first threshold (e.g., a distance of 10mm between the distal tip and the probe) may merely indicate that the probe 1140 has detected the magnetic field generated by the needle 1200 (e.g., wherein a corresponding alert may be a green indicator displayed by the console display 1130 and/or the probe 1140). However, upon the further advancement of the needle 1200 toward the probe 1140 and the crossing of a second threshold (e.g., a distance of 6mm between the distal tip and the probe), a second alert may be generated causing the green indicator to turn to an orange indicator. Similarly, upon further advancement of the needle 1200 toward the probe 1140 and the crossing of a third threshold (e.g., a distance of 2mm between the distal tip and the probe), a third alert may be generated causing the orange indicator to turn to a red indicator. As discussed above, the alerts may take several forms and the example above, such as with reference to specific colors, is not intended to limit the scope of the disclosure.

In some embodiments, the probe 1140 may include a processor (not shown) which may execute logic stored on non-transitory computer-readable memory included therein (not shown), such as the needle tracking logic 100. The needle tracking logic 100 may, upon execution, perform the same operations as discussed above with respect to the needle tracking logic 102. In some embodiments, indicator elements or a display screen of the probe 1140 (not shown) may be utilized to display visual alerts. In other embodiments, the determination of the position of the needle 1200 and/or the proximity of the needle 1200 to the probe 1140 may be transmitted to the console 1120, which subsequently determines the distance 400 is within one or more thresholds and generates a corresponding alert.

In some embodiments, the position of the needle 1200 in X, Y, and Z coordinate space with respect to the sensor array 1190 can be determined by the system 1110 using the magnetic field strength data sensed by the sensors 1192. Moreover, the pitch and yaw of the needle 1200 may also be determined. Suitable circuitry of the probe 1140, the console 1120, or other component of the system can provide the calculations necessary for such position/orientation. In one embodiment, the needle 1200 can be tracked using the teachings of one or more of the following U.S. patents: U.S. Patent No. 5,775,322; U.S. Patent No. 5,879,297; U.S. Patent No. 6,129,668; U.S. Patent No. 6,216,028; U.S. Patent No. 6,263,230; U.S. Patent No. 9,649,048; U.S. Patent No. 9,636,031; U.S. PatentNo. 9,554,716; U.S. Patent No. 9,521,961; U.S. Patent No. 9,492,097; U.S. Patent No. 9,456,766; U.S. Patent No. 8,849,382; U.S. Patent No. 8,781,555; U.S. PatentNo. 8,388,541; U.S. Patent No. 10,751,509; U.S. Patent No. 10,449,330; and U.S. Patent No. 10,524,694.

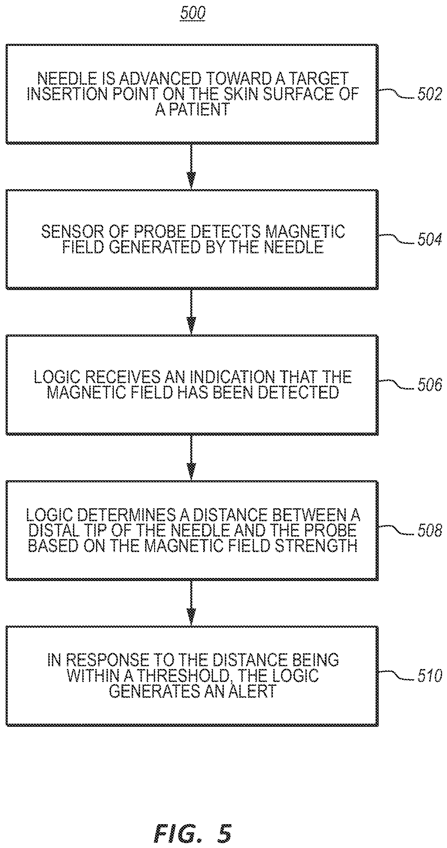

Referring to FIG. 5, a flowchart illustrating an exemplary method for detecting proximity of a needle to a probe using guidance system of FIG. 1 is shown according to some embodiments. Each block illustrated in FIG. 5 represents an operation performed in the method 500 of detecting proximity of a distal tip of a needle to a probe using guidance system of FIG. 1. Prior to detecting the proximity of the needle to the probe, in one embodiment, is assumed that the guidance system includes a probe having a body and a magnetic sensor, and the needle has been configured to generate a magnetic field (although other configurations for magnetic field generation may be deployed as discussed above). It is further assumed that the magnetic sensor is configured to detect the magnetic field generated by the needle. Finally, it is assumed that the guidance system includes logic that is stored on non-transitory computer-readable medium and, when executed by one or more processors, causes performance of operations associated with the proximity detection disclosed herein.

As an initial step in the method 500, the needle is advanced toward a target insertion point on the skin surface of a patient to access a target vessel with the needle by penetrating the skin surface (block 502). As is understood, the probe may be positioned on the skin surface enabling the projection of an ultrasound beam toward the target vessel for imaging purposes (see FIG. 4).

As the needle is advanced toward the target insertion, a sensor of the probe detects a magnetic field generated by or associated with the needle (block 504). Following detection of the magnetic field, logic of the guidance system receives an indication that the magnetic field has been detected (block 506). The indication includes a magnetic field strength. Subsequently, the logic determines a distance between the needle tip and the probe based at least in part on the magnetic field strength (block 508). As discussed above, the logic may be stored on a console, the probe or an alternative electronic device.

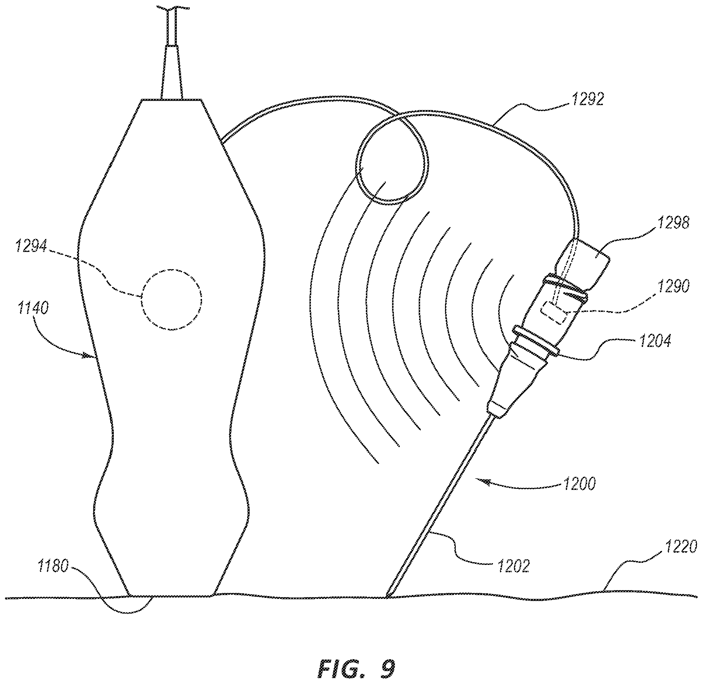

In some embodiments, depending on the positioning of the sensor in the probe, optionally the positioning of the magnetic elements associated with the needle, the length of the needle and/or the position of the magnetic sensor with respect to the distal end of the needle are input into or otherwise detectable or known by the guidance system and received by the logic. The length of the needle and/or the positioning of the magnetic sensor and elements may be provided when embodiments illustrated in FIGS. 9-10 are implemented. In some embodiments, as discussed above with respect to FIG. 4, position and orientation information of the needle maybe determined by the guidance system, which, together with the length of the cannula and, optionally, the position of the magnetic elements, enables the logic of the guidance system to accurately determine the location and orientation of the entire length of the needle with respect to the sensor array.

Finally, in response to determining the distance between the needle tip and the probe, the logic determines whether the distance is within a predefined threshold. When the distance is determined to be within the predefined threshold, the logic generates an alert, as discussed above (block 510).

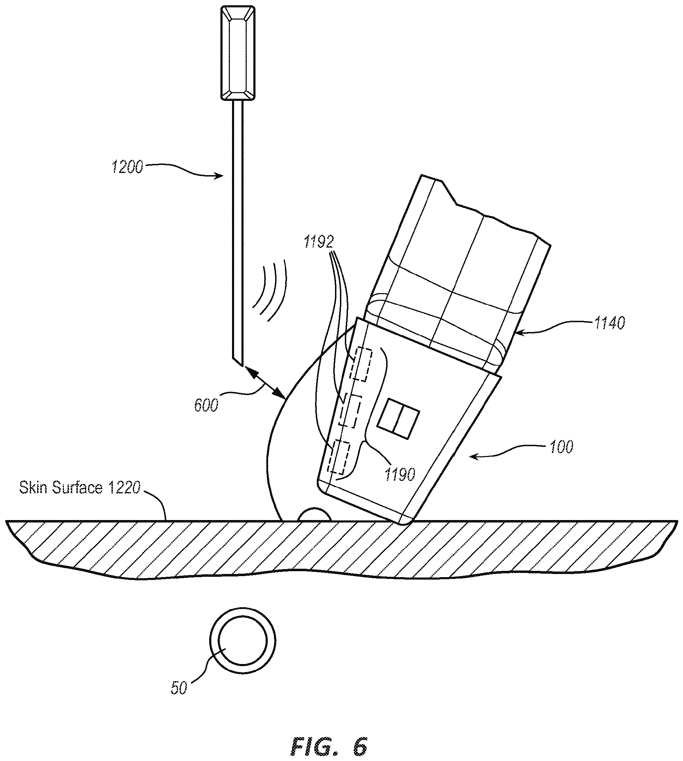

Referring to FIG. 6, is a second view of an ultrasound probe as a needle is advanced toward a target insertion site of a patient's skin is shown according to some embodiments. As shown in FIG. 6, a probe 1140 includes a probe head (e.g., a distal portion) that has installed thereon a needle shield 100. The needle shield 100 is coupled with the probe head, or portions thereof and cover a portion of the probe head. The needle shield 100 is formed of a resilient material that is resistant to penetrations from a needle. The needle shield 100 is formed of a material that is transparent to acoustic energy passing through the needle shield 100, either as transmitted energy from the transducer, or as reflected energy received by the transducer.

In an embodiment, the needle shield 100 is formed of a resilient material such as plastic, polymer, metal, or the like that is resistance to penetration from a needle 1200 and is substantially rigid. The needle shield 100 may define a substantially uniform thickness of between 0.25 mm to 5 mm, for example 1 mm. The needle shield conforms to the outer profile of the probe head and any associated covers, spacers, and the like to provide a protective layer thereover. As used herein, the needle shield 100 is described as co-operating with the probe head. However, it will be appreciated that the probe head can further include various covers, needle guides, spacers, and other additional structures. Accordingly, the needle shield 100 can be formed to co-operate with both the probe head and these additional structures, forming a protective barrier thereover. In an embodiment, the needle shield 100 is coupled with the probe head and secured thereto through mechanical interference against the probe head.

Although the needle shield 100 is formed of a resilient material configured to resist penetration from the needle, the guidance system disclosed herein may be configured to detect a magnetic field generated by the needle 1200 and is configured to determine the distance between a distal tip of the needle 1200 and the needle shield 100. The guidance system is further configured to generate an alert when the needle 1200 is advanced toward the skin surface such that the distal tip comes within a predefined distance of the needle shield 100.

In a similar manner as discussed above with respect to FIG. 4, the needle tracking logic 102 may, upon execution by the processor 1122, perform operations resulting in the determination of the position of the needle 1200; however, in this embodiment, the needle tracking logic 102 may determine the distance 600 between the distal tip of the needle 1200 and an exterior of the needle shield 100. As discussed above, the determination of the distance 600 may be optionally in conjunction with the length of the needle 1200 and the positioning of each sensor 1192 of the sensor array 1190 and based on the magnetic field strength detected by the sensor array 1190. The guidance system may be provided input regarding sizing information of the needle shield 100 or establish distance thresholds based on a default configuration (e.g., the needle shield extending beyond the exterior of the probe by 2mm).

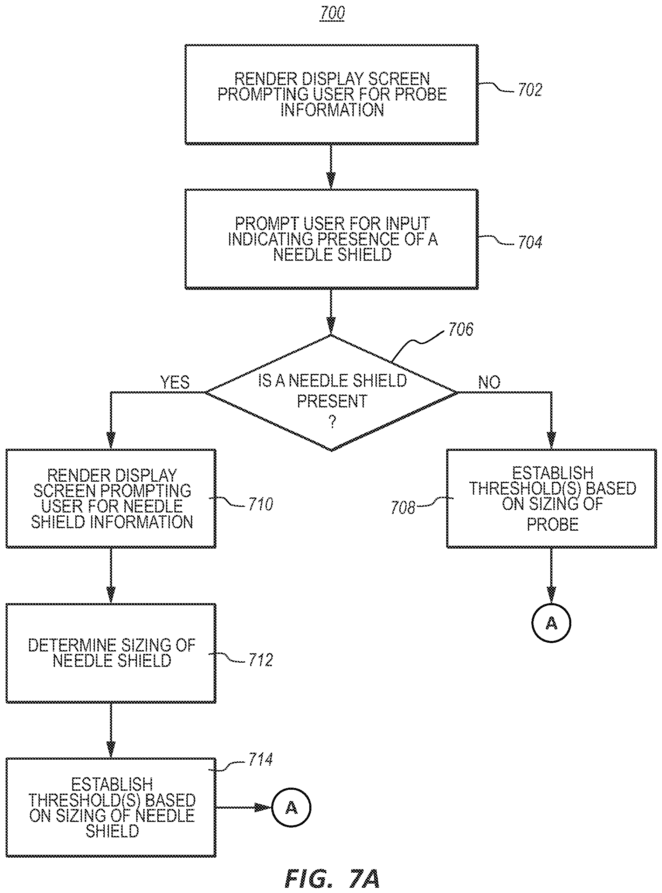

Referring to FIGS. 7A-7B, a flowchart illustrating an exemplary method for detecting proximity of a needle to a needle shield affixed to a probe using guidance system of FIG. 1 is shown according to some embodiments. Each block illustrated in FIGS. 7A-7B represents an operation performed in the method 700 of detecting proximity of a distal tip of a needle to a needle shield affixed to a probe using the guidance system of FIG. 1. Prior to detecting the proximity of the needle to the probe, in one embodiment, is assumed that the guidance system includes a probe having a body and a magnetic sensor, and the needle has been configured to generate a magnetic field (although other configurations for magnetic field generation may be deployed as discussed above). It is further assumed that the magnetic sensor is configured to detect the magnetic field generated by the needle. Finally, it is assumed that the guidance system includes logic that is stored on non-transitory computer-readable medium and, when executed by one or more processors, causes performance of operations associated with the proximity detection disclosed herein.

It is understood that one or more of the operations discussed below may be optional, and may not be included in certain embodiments. Referring to FIG. 7A, as an initial step in the method 700, a display screen is rendered that prompts the user for input pertaining to probe information (block 702). The probe information may be an indication as to a particular probe such as a product identifier.

An additional display screen may be rendered that prompts the user for input indicating the presence (or lack thereof) of a needle shield (block 704). Following receipt of input indicating the presence (or lack thereof) of the needle shield, the guidance system makes a determination as to the presence of the needle shield (block 706). When a needle shield is not present, the guidance system establishes one or more distance thresholds based on the sizing of the probe (block 708). In some embodiments, the sizing of the probe may be entered explicitly, for example via the display screen discussed with respect to block 702. Alternatively, when the input of block 702 is a product identifier, the guidance system may automatically determine the sizing information by querying a data store storing sizing information of a plurality of needle shields. For instance, the data store may store a configuration file that includes sizing information for a plurality of probes. Establishing the thresholds may include querying a data store for information pertaining to predefined thresholds, wherein one or more predefined thresholds are selected based on the sizing of the probe. Reference to a data store may refer to the same data store or one or multiple data stores included in or accessible to the guidance system. The method 700 then continues to block 716 discussed below.

When a needle shield is present, the guidance system renders a display screen that prompts the user for needle shield information (block 710). The needle shield information may correspond to sizing information of the needle shield or a product identifier such that the guidance system may query a data store for sizing information in a similar manner as discussed above with respect to the probe sizing information.

Following receipt of the needle shield information, the guidance system determines the sizing of the needle shield (block 712). As referenced above, the sizing of the needle shield may have been provided explicitly via the user input or determined automatically by the guidance system by querying a data store storing sizing information of a plurality of needle shields. Based on the sizing information of the needle shield, the guidance system establishes one or more distance thresholds (block 714). Establishing the thresholds may include querying a data store for information pertaining to predefined thresholds, wherein one or more predefined thresholds are selected based on the sizing of the needle shield. The method 700 then continues to block 716 discussed below.

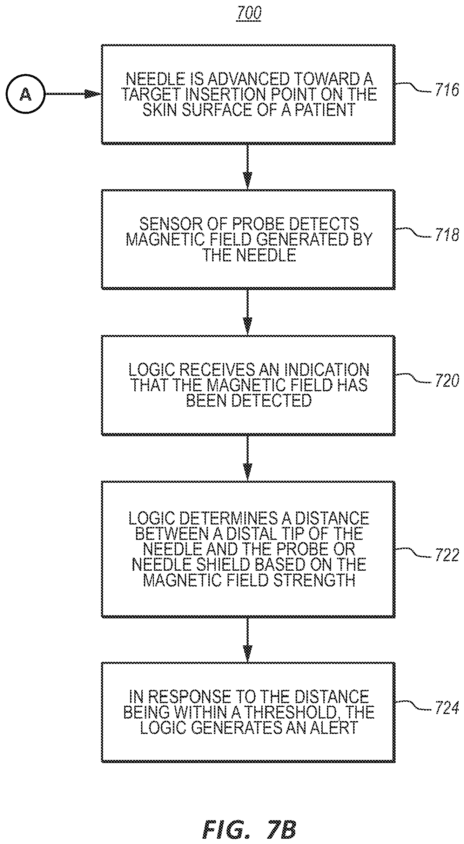

Referring now to FIG. 7B, the needle is advanced toward a target insertion point on the skin surface of a patient to access a target vessel with the needle by penetrating the skin surface (block 716). As the needle is advanced toward the target insertion, the sensor of the probe detects a magnetic field generated by or associated with the needle (block 718). Following detection of the magnetic field, logic of the guidance system receives an indication that the magnetic field has been detected (block 720). The indication includes a magnetic field strength. Subsequently, the logic determines a distance between the needle tip and the probe based at least in part on the magnetic field strength (block 722). When a needle shield is present, the logic of the guidance accounts for the sizing of the needle shield and determines a distance between the needle tip and the needle shield.

Finally, in response to determining the distance between the needle tip and the probe or the needle shield, the logic determines whether the distance is within a predefined threshold as previously established. When the distance is determined to be within the predefined threshold, the logic generates an alert, as discussed above (block 724).

Referring to FIG. 8, a side view of a needle for use with the guidance system of FIG. 1 is shown according to some embodiments. The illustration shows details of one example of the needle 1200 that can be used in connection with the guidance system 1110 in accessing a targeted internal body portion of the patient, as shown in FIG. 2, according to one embodiment. In particular, a magnetic element 1210 is included with the hub 1204 and may be a permanent magnet, including a ferromagnetic substance for instance. The magnetic element 1210 may be ring-shaped so as to define hole 1212 that is aligned with the hollow cannula 1202. So configured, the magnetic element 1210 generates a magnetic field that is detectable by the sensor array 1190 of the ultrasound probe 1140 so as to enable the location, orientation, and movement of the needle 1200 to be tracked by the system 1110, as described further herein.

Referring to FIGS. 9-10, simplified views of an ultrasound probe and needle including elements of an electromagnetic signal-based guidance system are shown according to some embodiments. The illustrations depict components of a guidance system according to one embodiment, wherein EM signal interaction between the probe 1140 and the needle 1200 is employed to enable tracking and guidance of the needle. In particular, in FIG. 9 the needle 1200 includes the stylet 1298 disposed therein, which includes an EM coil 1290 that is operably connected to the probe 1140 via a tether 1292. In this way, the EM coil 1290 can be driven by suitable components included in the probe 1140 or system console 1120 such that the EM coil emits an EM signal during operation.

A sensor 1294 suitable for detecting EM signals emitted by the EM coil 1290 of the stylet 1298 is included in the probe 1140. In the present embodiment, the sensor 1294 is a three-axis sensor for detecting corresponding orthogonal components of the EM signal, though other coil and sensor configurations can also be employed. So configured, the position and orientation of the needle 1200 can be determined, by EM signal triangulation or other suitable process, and displayed by the system in a manner similar to that already described above. As in previous embodiments, the processor 1122 of the system console 1120 (FIG. 1) can be employed to receive the sensed data of the EM sensor 1294 and compute the position and/or orientation of the needle 1200. As before, the length of the needle 1200 and/or the position of the EM coil 1290 with respect to the distal end of the needle 1200 are input into or otherwise detectable or known by the system.

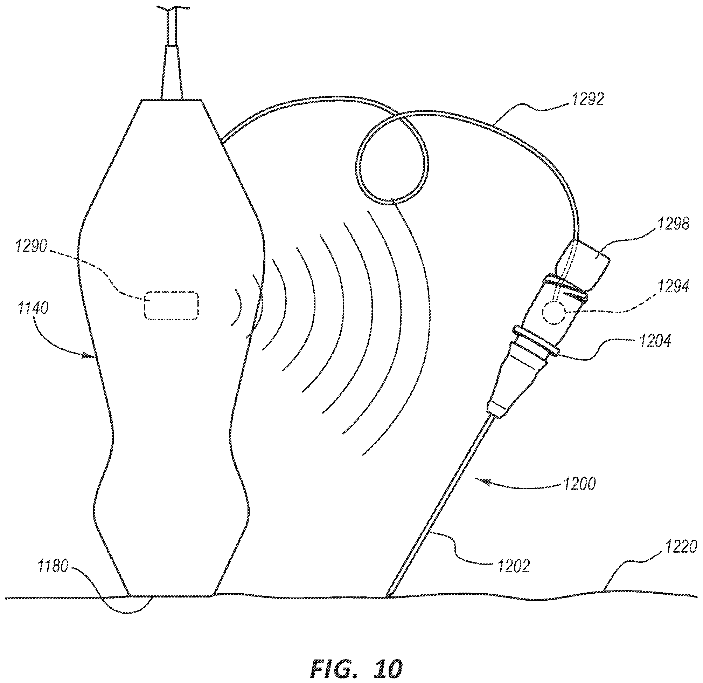

FIG. 10 shows a variation of the EM configuration of FIG. 9, wherein the respective positions of the EM components is reversed: the EM coil 1290 is included in the probe 1140 and the EM sensor 1294 is included with the stylet 1298 disposed in the needle 1200. Note that in the embodiments of FIGS. 9-10, the operable connection between the EM coil 1290 and the EM sensor 1294 via the tether 1292 enables the component disposed in the stylet 1298 to be driven by the system 1110. This also enables correspondence of the particular EM frequency/frequencies emitted by the EM coil 1290 and detected by the EM sensor 1294 to be made.

In one embodiment, the configurations shown in FIGS. 9-10 can be varied, wherein no tether operably connects the EM coil and the EM sensor; rather, the EM coil of the stylet operates as a separate component from the probe and its EM sensor and is powered by an independent power source, such as a battery. In this case, the probe/system includes suitable signal processing components configured to detect the EM signal emitted by the EM coil and to process it as necessary in order to locate the needle.

As disclosed herein, the guidance system disclosed may be configured to generate one or more alerts based on the proximity of a distal tip of a needle to a probe or needle shield. The alerts are intended to avoid penetration of the probe or needle shield by the needle. As a result, confidence may be maintained by a clinician and other medical professionals that the sterile field initially provided for the needle insertion process was preserved. Therefore, embodiments of the disclosure potentially eliminate the need for high level disinfection of the probe between each use.

While some particular embodiments have been disclosed herein, and while the particular embodiments have been disclosed in some detail, it is not the intention for the particular embodiments to limit the scope of the concepts provided herein. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the concepts provided herein.

## Claims

1. An ultrasound system (1110), comprising:
a probe (1140) including a probe head, a body, and a magnetic sensor (1192; 1294), the magnetic sensor configured to detect a magnetic field generated by or associated with a needle (1200);
a needle shield (100) coupled to the probe head, wherein the needle shield is formed of resilient material that is resistant to penetrations from a needle and is transparent to acoustic energy; and
logic stored on non-transitory computer-readable medium that, when executed by one or more processors, causes performance of operations including:
receiving (506) an indication that the magnetic field has been detected including a strength of the magnetic field,
determining (508) a distance between a distal tip of the needle and the needle shield based on the strength of the magnetic field, and
in response to the distance being within a first threshold, generating (510) a first alert.

2. The ultrasound system of claim 1, further comprising a console apparatus (1120) that includes the non-transitory computer-readable medium, the console apparatus communicatively coupled to the probe.

3. The ultrasound system of either claim 1 or claim 2, wherein the magnetic sensor includes a sensor array (1190) including a plurality of sensors.

4. The ultrasound system of any claim of claims 1-3, wherein the logic, when executed by the one or more processors, causes performance of further operations including determining a positioning of the needle in three spatial dimensions including X, Y, Z coordinate space.

5. The ultrasound system of claim 4, wherein the logic further detects a pitch attitude and a yaw attitude of the needle.

6. The ultrasound system of any claim of claims 1-5, wherein the non-transitory computer-readable medium is included within the probe.

7. The ultrasound system of any claim of claims 1-6, wherein the needle is magnetized and the magnetic field is generated by the magnetized needle.

8. The ultrasound system of any claim of claims 1-7, wherein the magnetic sensor is located at a distal end of the probe.

9. The ultrasound system of any claim of claims 1-8, wherein the magnetic sensor is a three-axis sensor (1294) configured to detect corresponding orthogonal components of the magnetic field.

10. A system comprising:
a needle (1200); and
the ultrasound system (1110) of any preceding claim.

11. The system of claim 10, wherein the needle comprises a magnetic element (1210) within a hub (1204) of the needle.

12. The system of claim 11, wherein the magnetic element is a permanent magnet.

13. The system of claim 11 or 12, wherein the magnetic element is ring-shaped so as to define a hole (1212) that is aligned with a hollow canula (1202) of the needle.

## Patentansprüche

1. Ultraschallsystem (1110), umfassend:
eine Sonde (1140) einschließlich eines Sondenkopfes, eines Körpers und eines Magnetsensors (1192; 1294), wobei der Magnetsensor so konfiguriert ist, dass er ein von einer Nadel (1200) erzeugtes oder mit ihr verbundenes Magnetfeld detektiert;
eine Nadelabschirmung (100), die mit dem Sondenkopf gekoppelt ist, wobei die Nadelabschirmung aus einem elastischen Material gebildet ist, das gegen das Eindringen einer Nadel resistent ist und für akustische Energie transparent ist; und
Logik, die auf einem nicht transitorischen computerlesbaren Medium gespeichert ist, die, wenn sie von einem oder mehreren Prozessoren ausgeführt wird, die Durchführung von Operationen bewirkt, einschließlich:
Empfangen (506) einer Anzeige, dass das Magnetfeld detektiert wurde, einschließlich einer Stärke des Magnetfelds,
Bestimmen (508) eines Abstands zwischen einer distalen Spitze der Nadel und der Nadelabschirmung basierend auf der Stärke des Magnetfelds, und
als Reaktion darauf, dass der Abstand innerhalb eines ersten Schwellenwertes liegt, Erzeugen (510) eines ersten Alarms.

2. Ultraschallsystem nach Anspruch 1, weiter umfassend eine Konsolenvorrichtung (1120), die das nicht transitorische computerlesbare Medium einschließt, wobei die Konsolenvorrichtung kommunikativ mit der Sonde gekoppelt ist.

3. Ultraschallsystem nach Anspruch 1 oder Anspruch 2, wobei der Magnetsensor ein Sensorarray (1190) einschließt, das eine Vielzahl von Sensoren einschließt.

4. Ultraschallsystem nach einem der Ansprüche 1-3, wobei die Logik, wenn sie von dem einen oder den mehreren Prozessoren ausgeführt wird, die Durchführung weiterer Operationen bewirkt einschließlich einer Bestimmung einer Positionierung der Nadel in drei räumlichen Dimensionen, einschließlich X, Y, Z-Koordinatenraum.

5. Ultraschallsystem nach Anspruch 4, wobei die Logik weiter eine Nicklage und eine Gierlage der Nadel detektiert.

6. Ultraschallsystem nach einem der Ansprüche 1-5, wobei das nicht transitorische computerlesbare Medium in der Sonde eingeschlossen ist.

7. Ultraschallsystem nach einem der Ansprüche 1-6, wobei die Nadel magnetisiert ist und das Magnetfeld durch die magnetisierte Nadel erzeugt wird.

8. Ultraschallsystem nach einem der Ansprüche 1-7, wobei der Magnetsensor an einem distalen Ende der Sonde angeordnet ist.

9. Ultraschallsystem nach einem Anspruch der Ansprüche 1-8, wobei der Magnetsensor ein Dreiachsensensor (1294) ist, der so konfiguriert ist, dass er entsprechende orthogonale Komponenten des Magnetfeldes detektiert.

10. System, umfassend:
eine Nadel (1200); und
das Ultraschallsystem (1110) nach einem vorstehenden Anspruch.

11. System nach Anspruch 10, wobei die Nadel ein magnetisches Element (1210) innerhalb einer Nabe (1204) der Nadel umfasst.

12. System nach Anspruch 11, wobei das magnetische Element ein Permanentmagnet ist.

13. System nach Anspruch 11 oder 12, wobei das magnetische Element ringförmig ist, um ein Loch (1212) zu definieren, das mit einer hohlen Kanüle (1202) der Nadel ausgerichtet ist.

## Revendications

1. Système à ultrasons (1110), comprenant :
une sonde (1140) incluant une tête de sonde, un corps, et un capteur (1192 ; 1294) magnétique, le capteur magnétique étant configuré pour détecter un champ magnétique généré par ou associé à une aiguille (1200) ;
un blindage (100) d'aiguille couplé à la tête de sonde, dans lequel le blindage d'aiguille est formé d'un matériau élastique qui est résistant vis-à-vis de pénétrations d'une aiguille et est transparent vis-à-vis d'une énergie acoustique ; et
une logique stockée sur un support non-transitoire lisible par ordinateur qui, lorsqu'elle est exécutée par un ou plusieurs processeurs, entraîne la mise en oeuvre d'opérations incluant :
la réception (506) d'une indication que le champ magnétique a été détecté incluant une intensité du champ magnétique,
la détermination (508) d'une distance entre une pointe distale de l'aiguille et le blindage d'aiguille sur la base de l'intensité du champ magnétique, et
en réponse au fait que la distance est à l'intérieur d'un premier seuil, la génération (510) d'une première alerte.

2. Système à ultrasons selon la revendication 1, comprenant en outre un appareil console (1120) qui inclut le support non-transitoire lisible par ordinateur, l'appareil console étant couplé en communication à la sonde.

3. Système à ultrasons selon la revendication 1 ou la revendication 2, dans lequel le capteur magnétique inclut un réseau (1190) de capteurs incluant une pluralité de capteurs.

4. Système à ultrasons selon l'une quelconque des revendications 1 à 3, dans lequel la logique, lorsqu'elle est exécutée par les un ou plusieurs processeurs, entraîne la mise en oeuvre d'autres opérations incluant la détermination d'un positionnement de l'aiguille dans trois dimensions spatiales incluant un espace de coordonnées X, Y, Z.

5. Système à ultrasons selon la revendication 4, dans lequel la logique détecte en outre une assiette en tangage et une assiette en lacet de l'aiguille.

6. Système à ultrasons selon l'une quelconque des revendications 1 à 5, dans lequel le support non-transitoire lisible par ordinateur est inclus dans la sonde.

7. Système à ultrasons selon l'une quelconque des revendications 1 à 6, dans lequel l'aiguille est magnétisée et le champ magnétique est généré par l'aiguille magnétisée.

8. Système à ultrasons selon l'une quelconque des revendications 1 à 7, dans lequel le capteur magnétique est situé à une extrémité distale de la sonde.

9. Système à ultrasons selon l'une quelconque des revendications 1 à 8, dans lequel le capteur magnétique est un capteur (1294) à trois axes configuré pour détecter des composantes orthogonales correspondantes du champ magnétique.

10. Système comprenant :
une aiguille (1200) ; et
le système à ultrasons (1110) selon l'une quelconque des revendications précédentes.

11. Système selon la revendication 10, dans lequel l'aiguille comprend un élément magnétique (1210) à l'intérieur d'un moyeu (1204) de l'aiguille.

12. Système selon la revendication 11, dans lequel l'élément magnétique est un aimant permanent.

13. Système selon la revendication 11 ou la revendication 12, dans lequel l'élément magnétique est en forme d'anneau de manière à définir un trou (1212) qui est aligné avec une canule creuse (1202) de l'aiguille.
